# EUROPEAN PATENT APPLICATION

(11) **EP 4 011 565 A1**
(43) Date of publication of application: **15.06.2022**
(21) Application number: 20863808.0
(22) Date of filing: 28.07.2020
(51) Int. Cl.: B25J 9/04, A61B 34/30

(54) **MEDICAL ARM SYSTEM, ARM DEVICE, AND MASTER-SLAVE SYSTEM OPERATION METHOD**

(30) Priority: 13.09.2019 JP 2019167725
(71) Applicant: Sony Group Corporation, Tokyo 108-0075 (JP)
(72) Inventor: WAKANA, Kazuhito, Tokyo 108-0075 (JP)
(74) Representative: D Young & Co LLP
(86) International application number: PCT/JP2020/028802
(87) International publication number: WO 2021/049189

(57) **Abstract**

A medical arm system (1) includes an operation apparatus (100) operated by an operator and an arm apparatus (50) remotely operated in response to an operation of the operator with respect to the operation apparatus (100), the arm apparatus (50) has a base (51), a first unit (60) connected to the base (51), a second unit (70) connected to the first unit (60), a gimbal (52) connected to the base (51) and supporting the second unit (70), and an end effector unit (80) connected to the second unit (70) and provided with an operation tool to contact a patient, the first unit (60) moves the second unit (70) in a direction of at least one axis with respect to the base (51), and the second unit (70) is interlocked with the first unit (60) in a state supported by the gimbal (52) and moves the end effector unit (80) in the direction of the at least one axis.

## Description

### Field

The present disclosure relates to a medical arm system, an arm apparatus, and an actuation method of a master/slave system.

### Background

Recently, operations using an operation robot of a master/slave type have been proposed. As an example of the operation robot of the master/slave type, there is an arm-type medical retention apparatus, in which medical optical equipment such as a camera head and a forward-oblique viewing endoscope is enabled to independently rotate about an optical axis (see Patent Literature 1), for example.

### Citation List

### Patent Literature

Patent Literature 1: JP 2019-84334 A

### Summary

### Technical Problem

However, in a case in which plural links are connected in a slave side by joint parts to function as one arm like the above described conventional art, it is difficult to enhance the rigidity of the arm since a point of support of the joint part and a point of action are distant from each other.

Therefore, the present disclosure proposes a medical arm system, an arm apparatus, and an actuation method of a master/slave system which enhance rigidity of an arm by a structure different from conventional arms.

### Solution to Problem

According to the present disclosure, a medical arm system includes: an operation apparatus operated by an operator; and an arm apparatus remotely operated in response to an operation of the operator with respect to the operation apparatus, wherein the arm apparatus has a base, a first unit connected to the base, a second unit connected to the first unit, a gimbal connected to the base and supporting the second unit, and an end effector unit connected to the second unit and provided with an operation tool to contact a patient, the first unit moves the second unit in a direction of at least one axis with respect to the base, and the second unit is interlocked with the first unit in a state supported by the gimbal and moves the end effector unit in the direction of the at least one axis.

Moreover, according to the present disclosure, an arm apparatus includes: a base; a first unit connected to the base; a second unit connected to the first unit; a gimbal connected to the base and supporting the second unit; and an end effector unit connected to the second unit and provided with an end effector to act on an object, wherein the first unit moves the second unit in a direction of at least one axis with respect to the base, and the second unit is interlocked with the first unit in a state supported by the gimbal and moves the end effector unit in the direction of the at least one axis.

### Brief Description of Drawings

FIG. 1 is a schematic diagram for describing outlines of a medical arm system according to a first embodiment of the present disclosure.
FIG. 2 is a schematic diagram illustrating an external configuration example of a slave apparatus according to the first embodiment.
FIG. 3 is an enlarged view of part of the slave apparatus according to the first embodiment.
FIG. 4 is an explanatory diagram illustrating an example of actions of an end effector unit of the slave apparatus according to the first embodiment.
FIG. 5 is an explanatory diagram illustrating examples of actions of an operation tool at a distal end of an end effector unit of the slave apparatus according to the first embodiment.
FIG. 6 is an explanatory diagram illustrating action examples of the slave apparatus according to the first embodiment.
FIG. 7A is a conceptual diagram illustrating a relation between a radius of an input-side capstan coupled to a first motor and a radius of an output-side capstan of a first unit.
FIG. 7B is an enlarged view of a part including the input-side capstan coupled to the first motor and the output-side capstan of the first unit.
FIG. 8A is a conceptual diagram illustrating a relation between a radius of an input-side capstan coupled to a second motor and a radius of an output-side capstan of a second unit.
FIG. 8B is an enlarged view of a part including the input-side capstan coupled to the second motor and the output-side capstan of the second unit.
FIG. 9 is a schematic diagram illustrating an external configuration example of a slave apparatus according to Modification Example 1.
FIG. 10A is a top view of the slave apparatus according to Modification Example 1.
FIG. 10B is a lateral view of the slave apparatus according to Modification Example 1.
FIG. 10C is a rear view of the slave apparatus according to Modification Example 1.
FIG. 11 is a schematic diagram illustrating an external configuration example of a slave apparatus according to Modification Example 2.
FIG. 12A is an explanatory diagram illustrating a state in which the end effector unit is placed on a slide base of the slave apparatus according to Modification Example 2.
FIG. 12B is an explanatory diagram illustrating disposition of the slide base of the slave apparatus according to Modification Example 2.
FIG. 13A is a top view of the slave apparatus according to Modification Example 2.
FIG. 13B is a lateral view of the slave apparatus according to Modification Example 2.
FIG. 13C is a rear view of the slave apparatus according to Modification Example 2.
FIG. 14 is a schematic diagram illustrating an external configuration example of a slave apparatus according to Modification Example 3.
FIG. 15 is a schematic diagram for describing outlines of a medical arm system according to a second embodiment of the present disclosure.
FIG. 16A is a schematic diagram illustrating an external configuration example of a slave apparatus according to the second embodiment.
FIG. 16B is an explanatory diagram for describing a first cable speed reducer of the slave apparatus according to the second embodiment.
FIG. 16C is an explanatory diagram for describing a second cable speed reducer of the slave apparatus according to the second embodiment.
FIG. 16D is an explanatory diagram for describing a slide mechanism of the slave apparatus according to the second embodiment.
FIG. 17A is a first perspective view of the slave apparatus according to the second embodiment.
FIG. 17B is a second perspective view of the slave apparatus according to the second embodiment.
FIG. 17C is a third perspective view of the slave apparatus according to the second embodiment.
FIG. 17D is a fourth perspective view of the slave apparatus according to the second embodiment.
FIG. 17E is a lateral view of the slave apparatus according to the second embodiment.
FIG. 18A is a rear view of a case in which two slave apparatuses according to the second embodiment are juxtaposed and used.
FIG. 18B is a first perspective view of a case in which the two slave apparatuses according to the second embodiment are juxtaposed and used.
FIG. 18C is a second perspective view of a case in which the two slave apparatuses according to the second embodiment are juxtaposed and used.
FIG. 18D is a lateral view of a case in which the two slave apparatuses according to the second embodiment are juxtaposed and used.
FIG. 18E is a top view of a case in which the two slave apparatuses according to the second embodiment are juxtaposed and used.

### Description of Embodiments

Hereinafter, embodiments of the present disclosure will be described in detail based on drawings. In following embodiments, the same parts are denoted by the same reference signs to omit redundant descriptions.

Also, the present disclosure is described in accordance with the order of items shown below.

### 1. First Embodiment

1.1. Outlines of Medical Arm System
1.1.1. Outlines of Master Apparatus
1.1.2. Outlines of Slave Apparatus
1.2. Details of Slave Apparatus
1.2.1. External Configuration Example of Slave Apparatus
1.2.2. Action Examples of Slave Apparatus
1.3. Modification Examples
1.3.1. Modification Example 1
1.3.2. Modification Example 2
1.3.3. Modification Example 3

### 2. Second Embodiment

2.1. Outlines of Medical Arm System
2.2. Details of Slave Apparatuses

### 3. Conclusion

### 1. First Embodiment

First, a medical arm system according to a first embodiment of the present disclosure will be described below in detail with reference to drawings. Note that, hereinafter, a medical robot system of a master/slave type is taken as an example to describe the medical arm system according to the first embodiment of the present disclosure.

### 1.1. Outlines of Medical Arm System

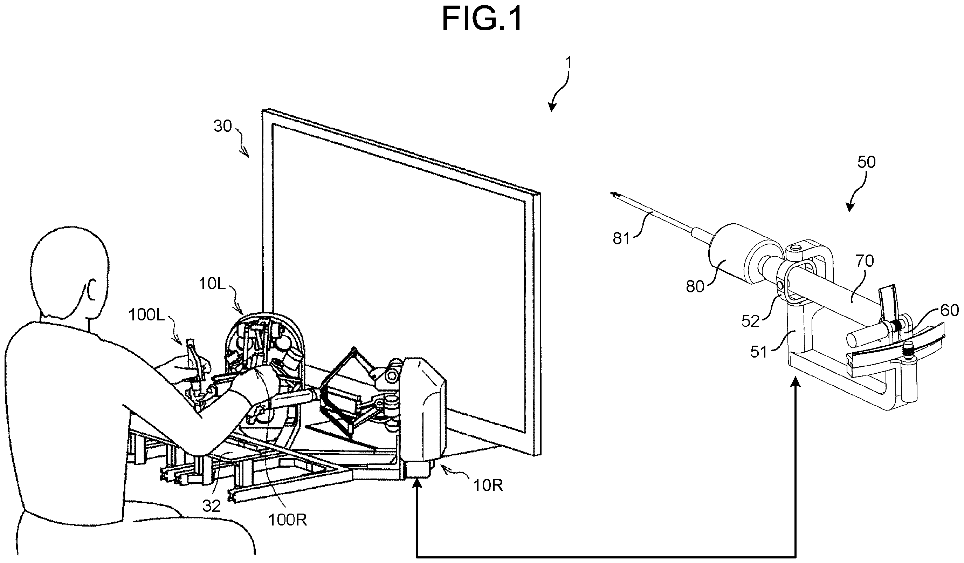

FIG. 1 is a schematic diagram for describing outlines of a medical arm system 1 according to the first embodiment. As illustrated in FIG. 1, the medical arm system 1 is provided with a master apparatus 10 (10R and 10L) and slave apparatuses 50. The master apparatus 10 is an apparatus provided with an input interface operated by an operator (hereinafter, also referred to as a user) such as a doctor. The slave apparatus 50 is an apparatus provided with a medical operation tool such as forceps or tweezers remotely operated in accordance with operations performed by the user of the master apparatus 10.

The medical arm system 1 employs, as an example, bilateral control. The bilateral control is feedback control that matches the positions and the state of force of the input interface and the operation tool between the master apparatus 10 and the slave apparatus 50. In other words, the bilateral control is the control that matches the positions and the state of force of the master apparatus and the slave apparatus at an arbitrary scale rate and also plays a role to transmit the positional change and force applied to the master apparatus by the user to an object. For example, when the user operates the input interface, the operation tool moves in accordance with the operation. When the operation tool moves and contacts a patient, the force upon the contact is fed back to the input interface.

Note that the master apparatus 10 and the slave apparatus 50 are connected to each other by an arbitrary communication method. For example, the master apparatus 10 and the slave apparatus 50 are connected to each other by wire communication or wireless communication. Also, for example, the master apparatus 10 and the slave apparatus 50 may be configured to carry out the communication directly or may be configured to carry out the communication via a network (or another apparatus).

### 1.1.1. Outlines of Master Apparatus

The master apparatus 10 is an information processing apparatus having functions of carrying out drive control of the slave apparatus 50 and presenting vibration signals (first signals), etc. measured by sensors of the slave apparatus 50 to the user.

As illustrated in FIG. 1, the master apparatus 10 is provided with operation apparatuses 100 (100R and 100L) held and operated by the user. The operation apparatuses 100 correspond to information processing apparatuses which transmit the sensations, which are produced when the operation tool of the slave apparatus 50 contacts an affected area or the like of the patient, to the user. In addition, a monitor 30 which displays an operative field is connected to the master apparatus 10, and the master apparatus 10 is provided with a support base 32 on which arms or elbows of the user are placed. Note that the master apparatus 10 includes a master apparatus 10R for the right hand and a master apparatus 10L for the left hand. Furthermore, the master apparatus 10R for the right hand is provided with the operation apparatus 100R for the right hand, and the master apparatus 10L for the left hand is provided with the operation apparatus 100L for the left hand.

The user places his/her arms or elbows on the support base 32 and holds the operation apparatuses 100R and 100L with the right hand and the left hand, respectively. In this state, the user operates the operation apparatuses 100R and 100L while watching the monitor 30, which displays the operative field. The user may remotely operate the positions or the directions of the operation tools attached to the slave apparatuses 50 or carry out holding actions by the respective operation tools by displacing the positions and the directions of the respective operation apparatuses 100R and 100L.

### 1.1.2. Outlines of Slave Apparatus

The slave apparatus 50 has a mechanism driven by an actuator such as a motor and moves in response to the drive control from the master apparatus 10. The slave apparatus 50 is an arm apparatus having a function of presenting the force and vibrations, which are generated when the affected area (hereinafter, will be also referred to as an object) of the patient in an operation and part of the slave apparatus 50, which is to contact the object, contact each other, to the master apparatus 10.

In the slave apparatus 50, an arm part 81, which contacts the object, is provided with various sensors (for example, an origin position sensor, a Limit sensor, an encoder, a microphone, an acceleration sensor, etc.). Also, the arm part 81 of the slave apparatus 50 is provided with a force sensor. The force sensor measures the force, which is applied to the arm part 81, when the operation tool at a distal end of the arm part 81 contacts the patient. Note that the locations at which the above described various sensors are provided are not particularly limited, and the various sensors may be provided at arbitrary locations of the arm part 81.

The slave apparatus 50 has, for example, a displacement sensor, which is for measuring the movement of a movable part (in other words, displacement of the position of the movable part), at a corresponding position. Examples of the above described displacement sensor include a potentiometer, an encoder, etc. Also, the slave apparatus 50 has, for example, a drive mechanism, which is for driving the above described movable part, at a position corresponding to the movable part. Examples of the above described drive mechanism include a motor, a driver thereof, etc.

### 1.2. Details of Slave Apparatus

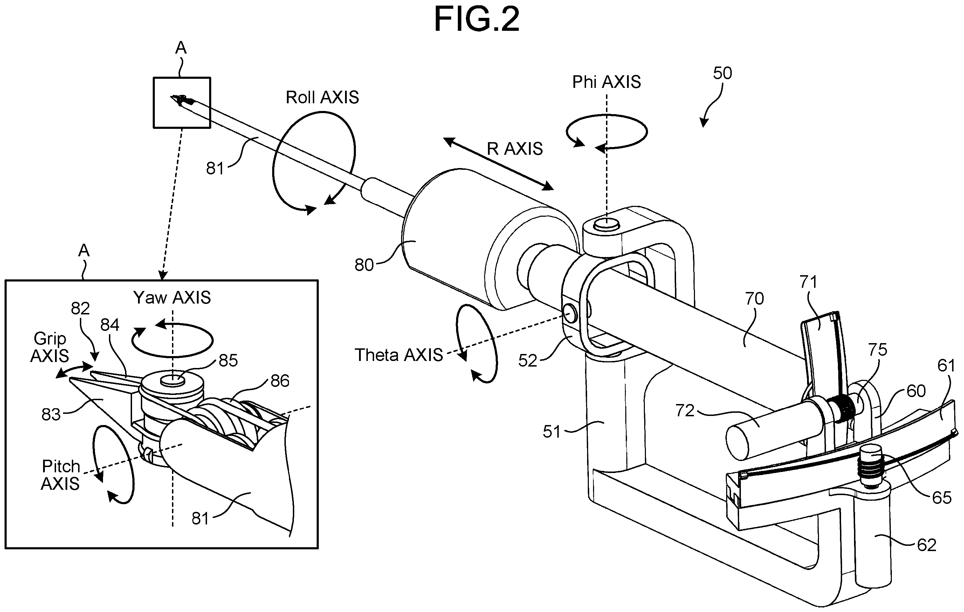

Hereinafter, the slave apparatus 50 according to the first embodiment will be described in more detail with reference to FIG. 2 to FIG. 8B.

### 1.2.1. External Configuration Example of Slave Apparatus

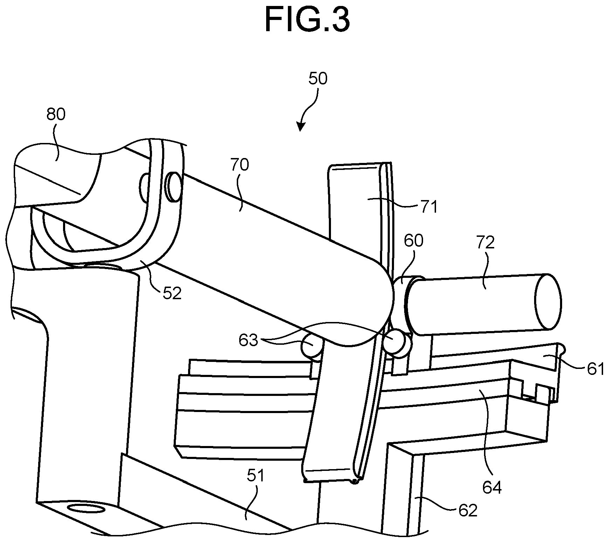

First, an external configuration example of the slave apparatus 50 will be described with reference to FIG. 2 and FIG. 3. FIG. 2 is a schematic diagram illustrating the external configuration example of the slave apparatus according to the first embodiment. FIG. 3 is an enlarged view of part of the slave apparatus according to the first embodiment.

As illustrated in FIG. 2, the slave apparatus 50 is provided with a base 51, a gimbal 52, a first unit 60, a first cable speed reducer 61, a first motor 62, a second unit 70, a second cable speed reducer 71, a second motor 72, and an end effector unit 80.

The gimbal 52 and the first unit 60 are connected to the base 51. The second unit 70 is connected to the first unit 60. The second unit 70 is a long part, is supported by the gimbal 52, which carries out rotation support of two axes, and carries out actions of two degrees of freedom in a polar coordinate system by the first motor 62 and the second motor 72 disposed at the positions distant from a rotation center of the gimbal 52.

In the present embodiment, the first unit 60 carries out arc motions about a Phi axis of the gimbal 52 together with the second unit 70 by the rotation of the first motor 62 via the first cable speed reducer 61. Also, the second unit 70 carries out arc motions about a Theta axis of the gimbal 52 by the rotation of the second motor 72 via the second cable speed reducer 71. Each of the first cable speed reducer 61 and the second cable speed reducer 71 carries out speed reduction by using at least one cable (wire). Also, as illustrated in FIG. 2, the gimbal 52 has a hollow center and has a shape (center hollow shape) double supported respectively in a Phi-axis direction and a Theta-axis direction.

Herein, an arc guide 64 illustrated in FIG. 3 is an arc-shaped guide part, and the first unit 60 can smoothly carry out an arc motion about the Phi axis of the gimbal 52 by using this. Part of the first unit 60 also works as an output-side capstan of the first cable speed reducer 61 and acts in response to torque from the first motor 62 fixed to the base 51. Also, a power transmission part 65 coupled to the first motor 62 works as an input-side capstan.

As well as the first unit 60, an end (first end) of one side of the second unit 70 also works as an output-side capstan of the second cable speed reducer 71 and acts in response to torque from the second motor 72 fixed to the first unit 60. Also, a power transmission part 75 coupled to the second motor 72 works as an input-side capstan.

As illustrated in FIG. 3, right/left rattling of both edges of the output-side capstan (second cable speed reducer 71) of the second unit 70 with respect to the first unit 60 is regulated by cam followers 63 fixed to the first unit 60 so that only arc motions about the Theta axis can be carried out. The cam follower 63 is a shaft-equipped bearing having a thick outer ring and having high rigidity with a needle-like roller called a needle built therein.

In the slave apparatus 50, using the cable speed reducers (the first cable speed reducer 61 and the second cable speed reducer 71) with respect to the Phi axis and the Theta axis is an effective means to realize backlashless and improve back-drivability. As a matter of course, power transmission by gear may also be used.

Furthermore, the slave apparatus 50 according to the first embodiment can realize 7-axis drive as illustrated in FIG. 2 by attaching the end effector unit 80 to the other end (second end) of the second unit 70.

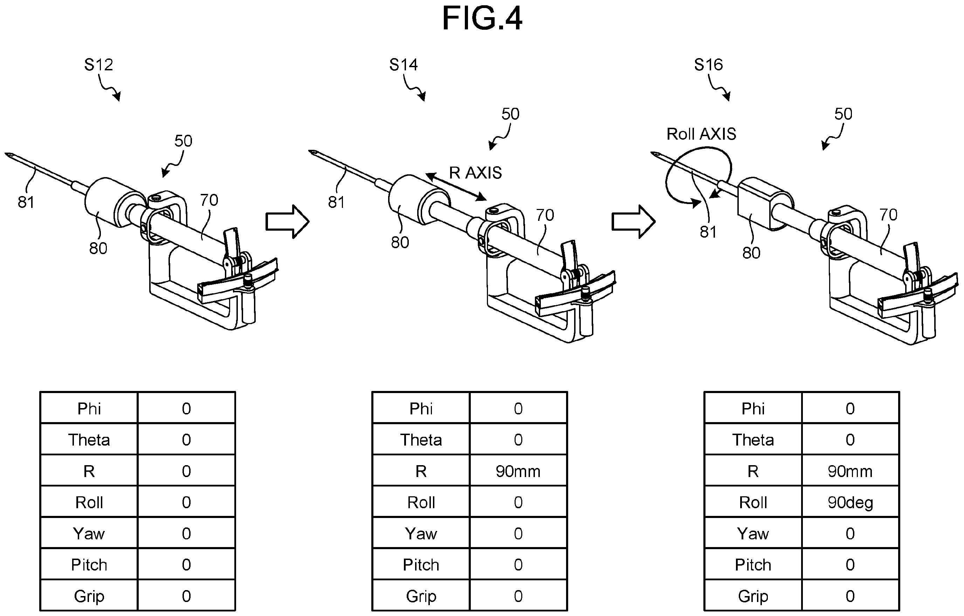

The end effector unit 80 is an operation tool unit, carries out slide motions in a long axis direction with respect to the second unit 70, and carries out Roll axis rotations about the long axis of the second unit 70. For example, the end effector unit 80 extends by 90 mm in an R-axis direction as illustrated in step S12 to step S14 of FIG. 4 and rotates by 90 degrees in the rotation direction of a Roll axis as illustrated from step S14 to step S16. Note that numerical values such as angles illustrated in FIG. 4 represent the amounts of changes with respect to an initial state (reference position).

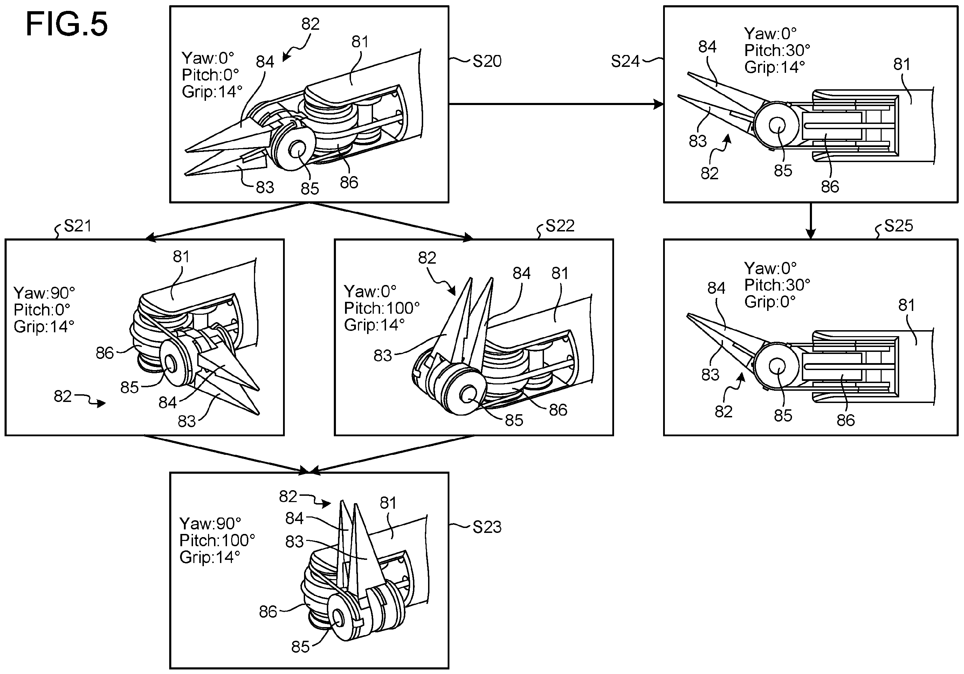

Furthermore, as illustrated in FIG. 2, the slave apparatus 50 according to the first embodiment can carry out 3-axis motions of Yaw-axis/Pitch-axis/Grip-axis with a distal end portion A of the arm part 81 of the end effector unit 80. In the present embodiment, the distal end portion A of the arm part 81 of the end effector unit 80 is provided with a gripper 82, which can be used as an operation tool such as forceps or tweezers, as an example of an end effector which acts on the object. The gripper 82 is formed by a first blade 83, a second blade 84, a first rotation shaft 85, and a second rotation shaft 86. For example, the state illustrated in step S20 of FIG. 5 is an initial state of the gripper 82. As illustrated in step S20 to step S21 and/or step S22 to step S23 of FIG. 5, the entirety of the first blade 83, the second blade 84, and the first rotation shaft 85 moves in the rotation direction of the Yaw axis (in this case, by 90 degrees) while the second rotation shaft 86 serves as a point of support. Also, as illustrated in step S20 to step S22 and/or step S21 to step S23 of FIG. 5, the entirety of the first blade 83 and the second blade 84 moves in the rotation direction of the Pitch axis (in this case, by 100 degrees) while the first rotation shaft 85 serves as a point of support. Furthermore, even in a state in which the gripper 82 has been moved in the rotation direction of the Pitch axis (in this case, moved by 30 degrees) as illustrated in step S20 to step S24 of FIG. 5, the first blade 83 and the second blade 84 opens/closes in the rotation direction of the Grip axis while the first rotation shaft 85 serves as a point of support as illustrated in step S24 to step S25 of FIG. 5. As a matter of course, an action in the opposite direction, for example, an action of returning to the original state can be also carried out. Note that the numerical values such as angles illustrated in FIG. 5 represent the amounts of changes with respect to the initial state (reference position).

Regarding the Yaw axis, the Pitch axis, and the Grip axis, for example, actions are carried out by transmitting the force of plural actuators disposed at a root (in the side of the second unit 70) of the end effector unit 80 to the gripper 82 provided at the distal end portion A of the arm part 81 by wire ropes.

### 1.2.2. Action Examples of Slave Apparatus

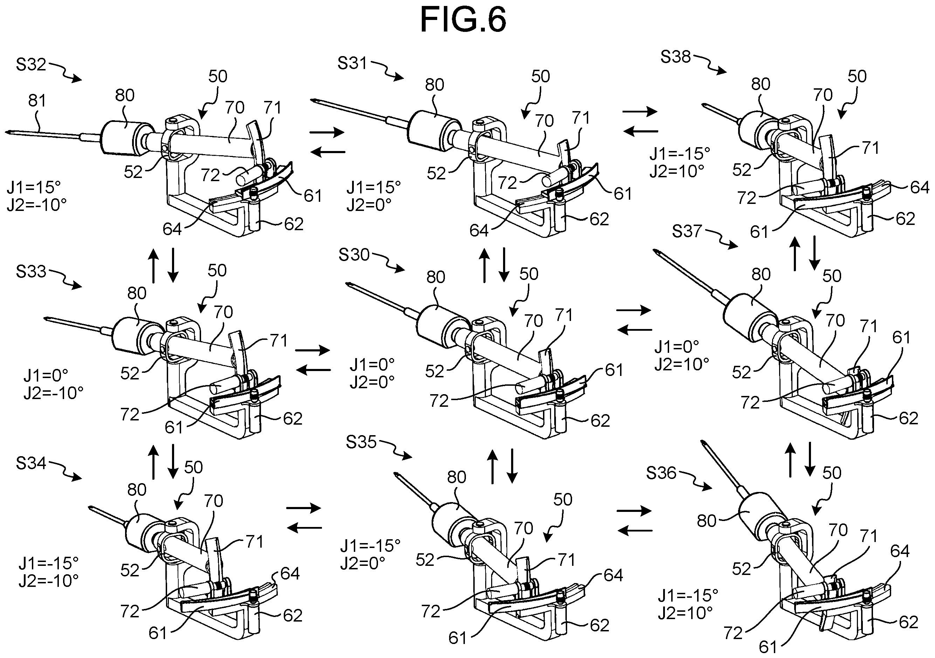

Next, action examples of the slave apparatus 50 will be described with reference to FIG. 6 to FIG. 8B. FIG. 6 is an explanatory diagram illustrating action examples of the slave apparatus 50 according to the first embodiment. FIG. 6 illustrates states of the mechanism of the slave apparatus 50 when polar coordinate system actions (arc motions) in the Phi axis (Joint1=J1) and the Theta axis (Joint2=J2) are carried out. Note that the numerical values such as angles illustrated in FIG. 6 represent the amounts of changes with respect to the initial state (reference position). The state (J1=0 degree, J2=0 degree) illustrated in step S30 of FIG. 6 is assumed to be the initial state of the slave apparatus 50 serving as a reference. When movement is carried out by 15 degrees in the rotation direction of the Phi axis in the state illustrated in step S30, the state illustrated in step S31 (J1=15 degrees, J2=0 degree) is obtained. When movement is carried out by -10 degrees in the rotation direction of the Theta axis in the state illustrated in step S31, the state illustrated in step S32 (J1=15, J2=-10 degrees) is obtained. When movement is carried out by -15 degrees in the rotation direction of the Phi axis in the state illustrated in step S32, the state illustrated in step S33 (J1=0, J2=-10 degrees) is obtained. When movement is carried out by -15 degrees in the rotation direction of the Phi axis in the state illustrated in step S33, the state illustrated in step S34 (J1=-15 degrees, J2=-10 degrees) is obtained. When movement is carried out by 10 degrees in the rotation direction of the Theta axis in the state illustrated in step S34, the state illustrated in step S35 (J1=-15 degrees, J2=0 degree) is obtained. When movement is carried out by 10 degrees in the rotation direction of the Theta axis in the state illustrated in step S35, the state illustrated in step S36 (J1=-15 degrees, J2=10 degrees) is obtained. When movement is carried out by 15 degrees in the rotation direction of the Phi axis in the state illustrated in step S36, the state illustrated in step S37 (J1=0, J2=10 degrees) is obtained. When movement is carried out by -15 degrees in the rotation direction of the Phi axis in the state illustrated in step S37, the state illustrated in step S38 (J1=-15 degrees, J2=10 degrees) is obtained. Note that, when movement is carried out by 30 degrees in the rotation direction of the Phi axis and movement is carried out by -10 degrees in the rotation direction of the Theta axis in the state illustrated in step S38, the state illustrated in step S31 (J1=15 degrees, J2=0 degree) is obtained. Also, when movement is carried out by -15 degrees in the rotation direction of the Phi axis in the state illustrated in step S30, which is the initial state, the state illustrated in step S35 (J1=-15 degrees, J2=0 degree) is obtained. When movement is carried out by -10 degrees in the rotation direction of the Theta axis in the state illustrated in step S30, the state illustrated in step S33 (J1=0, J2=-10 degrees) is obtained. When movement is carried out by 10 degrees in the rotation direction of the Theta axis in the state illustrated in step S30, the state illustrated in step S37 (J1=0, J2=10 degrees) is obtained. As a matter of course, an action in the opposite direction, for example, an action of returning to the original state can be also carried out. As illustrated in FIG. 6, the slave apparatus 50 according to the first embodiment can realize actions in polar coordinates without interference between the action in the Phi axis and the action in the Theta axis.

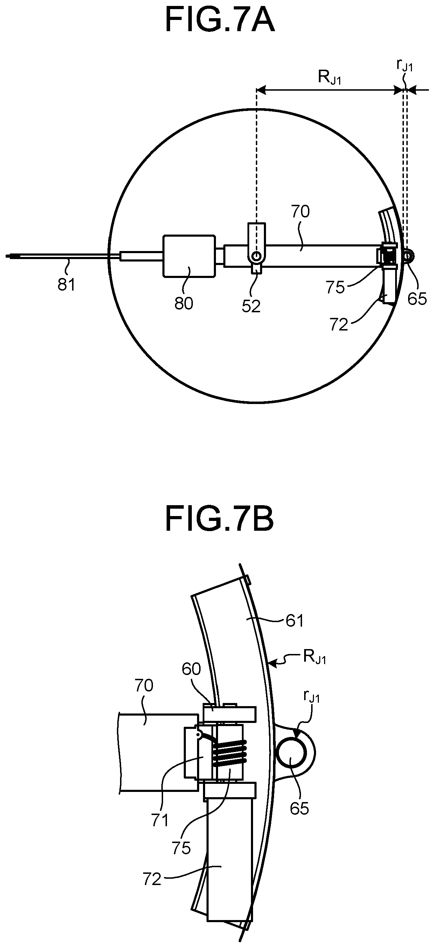

FIG. 7A and FIG. 7B are explanatory diagrams illustrating a speed reducing ratio in the Phi axis (Joint1=J1) of the slave apparatus 50 according to the first embodiment. FIG. 7A is a conceptual diagram illustrating a relation between a radius r_{J1} of the input-side capstan coupled to the first motor 62 and a radius R_{J1} of the output-side capstan of the first unit 60. FIG. 7B is an enlarged view of a part including the input-side capstan coupled to the first motor 62 and the output-side capstan of the first unit 60. The speed reducing ratio in the Phi axis is obtained by r_{J1}/R_{J1} (=2πr_{J1}/2πR_{J1}) by using the radius r_{J1} of the input-side capstan coupled to the first motor 62 and the radius R_{J1} of the output-side capstan of the first unit 60.

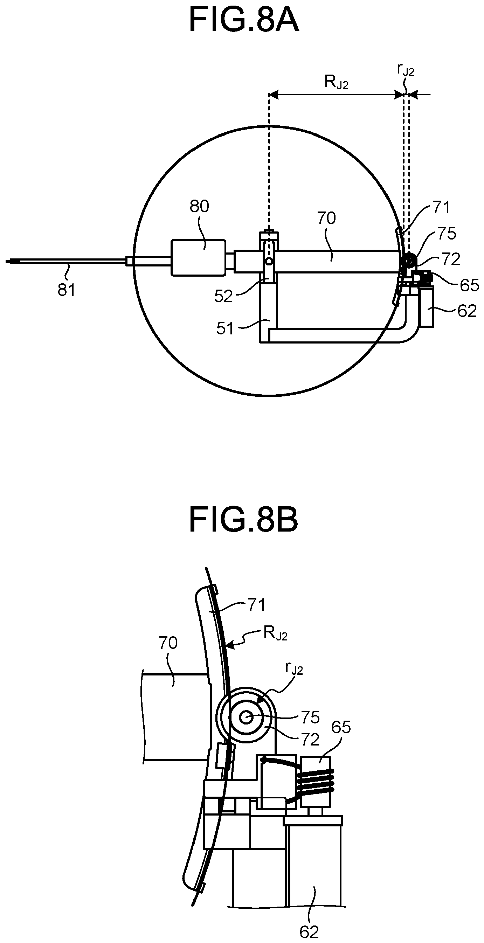

FIG. 8A and FIG. 8B are explanatory diagrams illustrating a speed reducing ratio in the Theta axis (Joint2=J2) of the slave apparatus 50 according to the first embodiment. FIG. 8A is a conceptual diagram illustrating a relation between a radius r_{J2} of the input-side capstan coupled to the second motor 72 and a radius R_{J2} of the output-side capstan of the second unit 70. FIG. 8B is an enlarged view of a part including the input-side capstan coupled to the second motor 72 and the output-side capstan of the second unit 70. The speed reducing ratio in the Theta axis is obtained by r_{J2}/R_{J2}(=2πr_{J2}/2πR_{J2}) by using the radius r_{J2} of the input-side capstan coupled to the second motor 72 and the radius R_{J2} of the output-side capstan of the second unit 70.

As described above, the slave apparatus 50 according to the first embodiment is an arm apparatus having an oar mechanism, which has a configuration of degrees of freedom of a polar coordinate type, and carries out polar-coordinate-system actions in the Phi axis and the Theta axis about the gimbal 52, which supports rotation in the two axes. Specifically, the slave apparatus 50 according to the first embodiment includes seven axes in total, i.e., three axes of operation-tool-position changing actions in the Phi axis, the Theta axis, and the R axis, three axes of operation-tool rotating actions in the Yaw axis, the Pitch axis, and the Roll axis, and one axis of an operation-tool opening/closing action in the Grip axis. The mechanism which constitutes the three axes of the operation-tool-position changing actions is referred to as an oar mechanism, and the three degrees of freedom at the distal end (actions in the Yaw axis, the Pitch axis, and the Grip axis) are realized by a wire towing mechanism. Also, parallel drive in the R axis and the Yaw axis can be also carried out.

A reason why the above described mechanism constituting the three axes of the operation-tool-position changing actions is called an oar mechanism is that the behavior of applying torque to an end part of the long mechanism about the gimbal 52 and carrying out polar-coordinate-system actions in the Phi axis and the Theta axis is similar to motions of an oar of a boat. In this oar mechanism, since force is applied at a position distant from a rotation center, high speed reducing ratios can be obtained (high output) even with the cable speed reducers, and high rigidity can be ensured (high rigidity) even with wire drive, which has comparatively low rigidity. Also, since the mechanism is backlashless and has high back-drivability, the mechanism is effective for smooth and fine locating actions (high precision). Furthermore, since a vicinity of the gravity center of the mechanism is supported by the gimbal 52, gravity compensating torque required for the motors can be reduced (gravity compensation).

Specifically, the slave apparatus 50 according to the first embodiment has the second unit 70 supported by a rotation base (gimbal 52) and can carry out actions of two degrees of freedom in the Phi axis and the Theta axis (operation-tool-position changing actions in two axes). The second unit 70 is connected to the first unit 60. The first unit 60 acts along an arc-shaped trajectory about the Phi axis. The second unit 70 obtains rotative power about the Phi axis from the first unit 60 and carries out actions in the Phi axis. Furthermore, in addition to the Phi-axis actions, the second unit 70 obtains rotative power about the Theta axis and carries out Theta-axis actions. As a result, the second unit 70 carries out two-axis operation-tool-position changing actions of Phi-axis actions and Theta-axis actions.

The second unit 70 is connected to/equipped with the end effector unit 80. The end effector unit 80 can carry out R-axis actions (slide motions in the long axis direction), which enable forward/backward movement in the longitudinal direction of the second unit 70 with respect to the second unit 70, and carry out Roll-axis actions (rotation motions about the long axis), which enable rotation about the longitudinal-direction axis of the second unit 70. The R-axis actions are operation-tool-position changing actions, and the Roll-axis actions are operation-tool rotating actions. The R-axis actions and the Roll-axis actions can be also carried out in the end effector unit 80. Also, in practice, the R-axis actions may be realized by expansion-contraction/slide motions of at least part of the second unit 70. Similarly, the Roll-axis actions may be realized by rotation motions of at least part of the second unit 70 about the long axis. The gripper 82 provided at the distal end of the arm part 81 of the end effector unit 80 can carry out operation-tool rotating actions in the two axes of the Pitch axis and the Yaw axis and carry out operation-tool opening/closing actions in the Grip axis.

### 1.3. Modification Examples

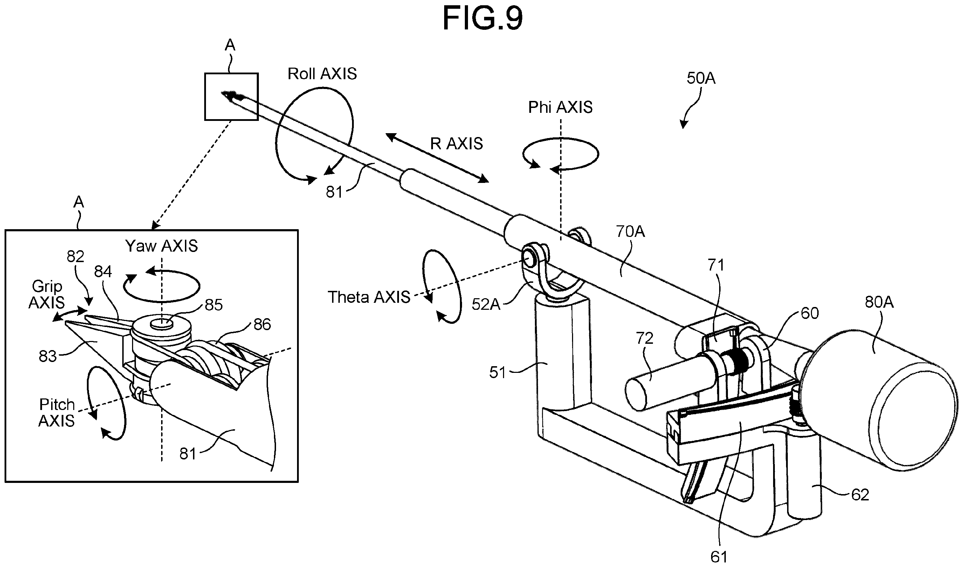

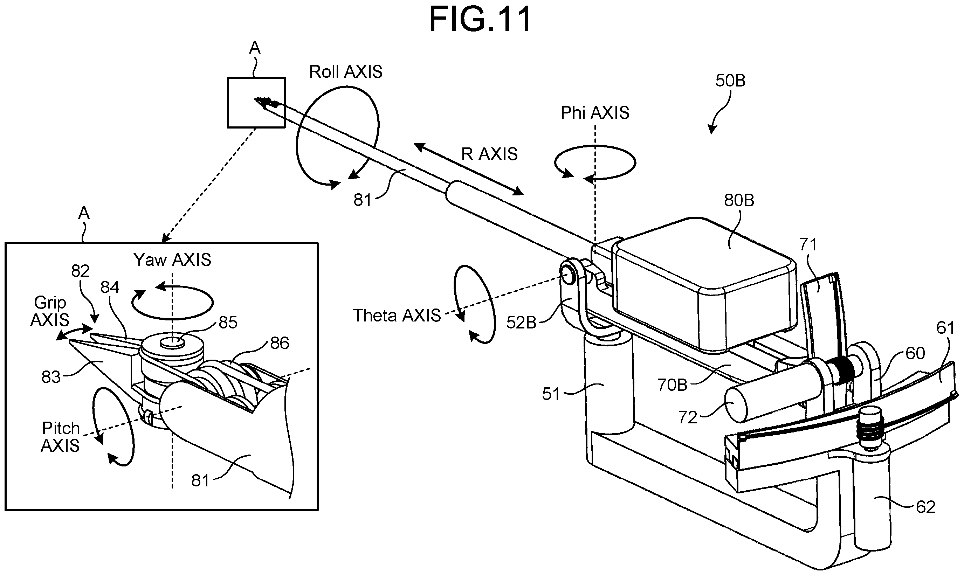

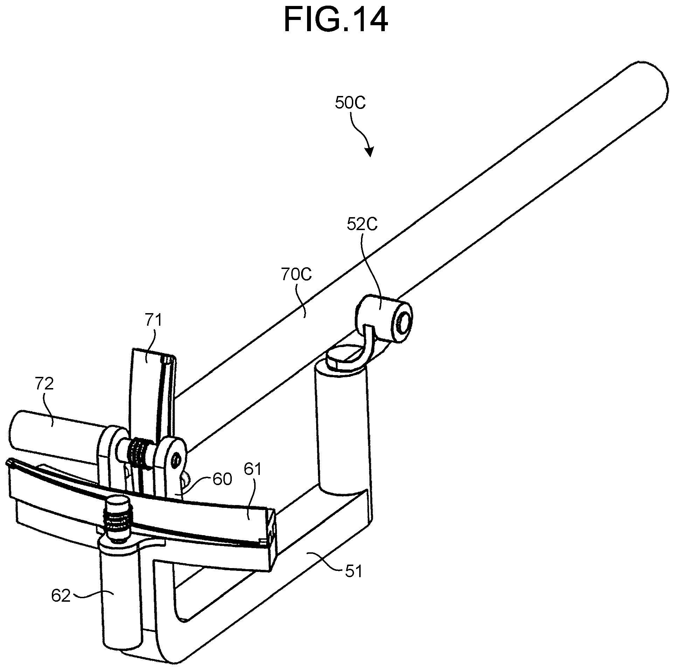

Next, modification examples of the slave apparatus 50 according to the first embodiment of the present disclosure will be described with reference to FIG. 9 to FIG. 14. FIG. 9 to FIG. 10C are diagrams for describing a slave apparatus 50A according to Modification Example 1. FIG. 11 to FIG. 13C are diagrams for describing a slave apparatus 50B according to Modification Example 2. FIG. 14 is a diagram for describing a slave apparatus 50C according to Modification Example 3.

### 1.3.1. Modification Example 1

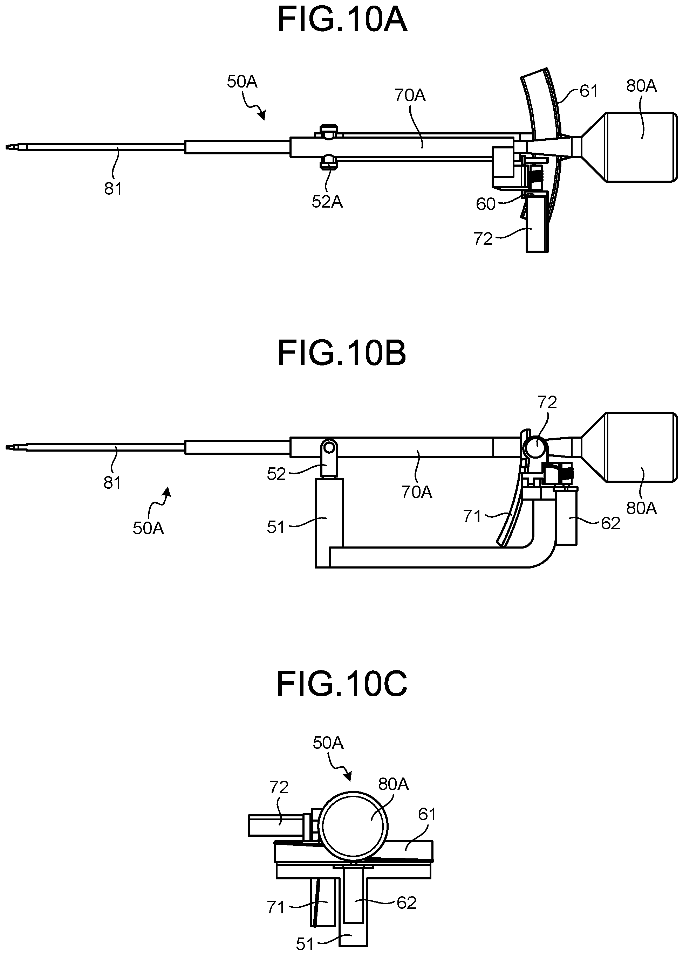

FIG. 9 is a schematic diagram illustrating an external configuration example of the slave apparatus 50A according to Modification Example 1. FIG. 10A is a top view of the slave apparatus 50A according to Modification Example 1. FIG. 10B is a lateral view of the slave apparatus 50A according to Modification Example 1. FIG. 10C is a rear view of the slave apparatus 50A according to Modification Example 1. As illustrated in FIG. 9 and FIG. 10A to FIG. 10C, a gimbal 52A may be a cantilever in the Phi-axis direction and have a double-supported shape (U shape) in the Theta-axis direction. Also, conversely, the gimbal 52A may be double-supported in the Phi-axis direction and have a cantilever shape (lateral U shape (C shape)) in the Theta-axis direction. Also, since the second cable speed reducer 71 is laterally shifted from the long axis of a second unit 70A, an end effector unit 80A can be connected from the rear of the second unit 70A. In this case, load on the first motor 62 and the second motor 72 increases since the positions of gravity centers of the second unit 70A and the end effector unit 80A become distant from the gimbal 52A. However, this structure does not easily interfere with a work object since the drive mechanism of the gripper 82 is not required to be disposed in the vicinity of the distal end of the arm part 81.

### 1.3.2. Modification Example 2

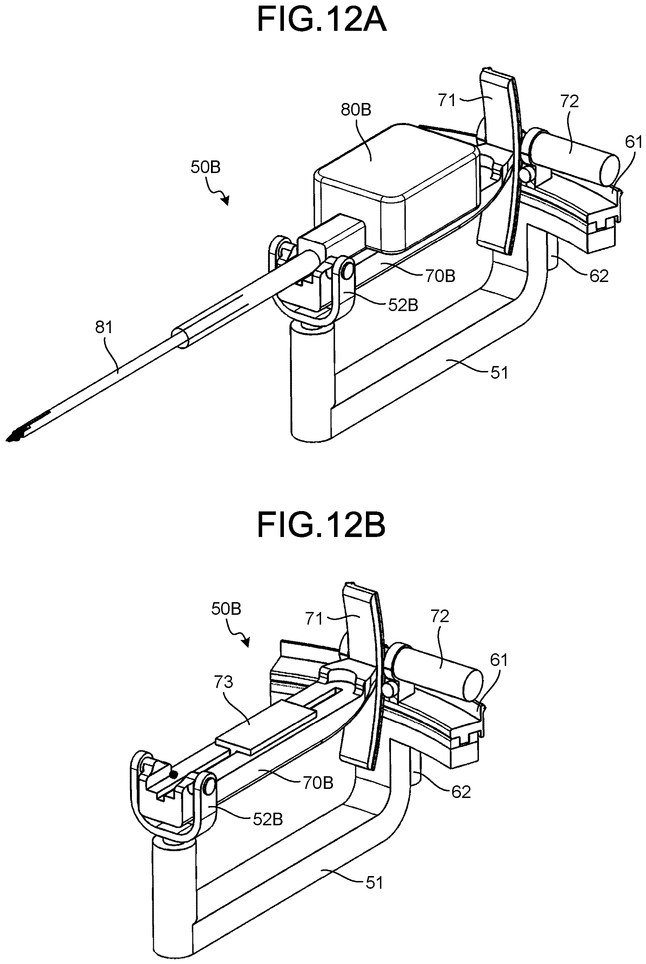

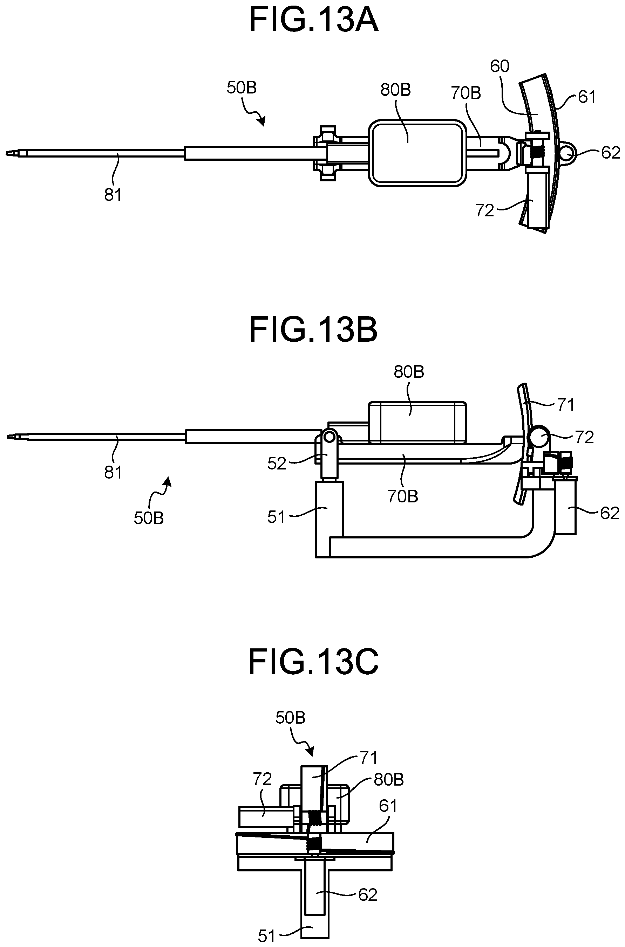

FIG. 11 is a schematic diagram illustrating an external configuration example of the slave apparatus 50B according to Modification Example 2. FIG. 12A is an explanatory diagram illustrating a state in which the end effector unit is placed on a slide base of a slave apparatus according to Modification Example 2. FIG. 12B is an explanatory diagram illustrating disposition of the slide base of the slave apparatus according to Modification Example 2. FIG. 13A is a top view of the slave apparatus 50B according to Modification Example 2. FIG. 13B is a lateral view of the slave apparatus 50B according to Modification Example 2. FIG. 13C is a rear view of the slave apparatus 50B according to Modification Example 2. As illustrated in FIG. 11 to FIG. 13C, since a gimbal 52B is configured to have a cantilever shape (U shape) in the Phi-axis direction, an upper portion of a second unit 70B can be configured to be in an open state. Also, as illustrated in FIG. 12A and FIG. 12B, a slide base 73, which is movable in the R-axis direction, is disposed at the location where the upper portion is open, and an end effector unit 80B is configured to be placed on the slide base 73. The slave apparatus 50B according to Modification Example 2 is capable of configuring a gimbal rear side to be compact as well as Modification Example 1 and disposing the gravity center position further closer to the gimbal compared with Modification Example 1.

### 1.3.3. Modification Example 3

FIG. 14 is a schematic diagram illustrating an external configuration example of the slave apparatus 50C according to Modification Example 3. As illustrated in FIG. 14, a gimbal 52C may have a cantilever structure (L shape) both in the Phi axis and the Theta axis. In FIG. 14, illustration of the end effector unit 80 is omitted. The shape of a second unit 70C illustrated in FIG. 14 may be the same as that of the second unit 70 of the first embodiment, the second unit 70A of Modification Example 1, or the second unit 70B of Modification Example 2.

### 2. Second Embodiment

Next, a medical arm system 1 according to a second embodiment of the present disclosure will be described below in detail with reference to drawings. The medical arm system 1 according to the second embodiment corresponds to an example of the medical arm system 1 according to the first embodiment. Note that, hereinafter, a medical robot system of a master/slave type is taken as an example to describe the medical arm system according to the second embodiment.

### 2.1. Outlines of Medical Arm System

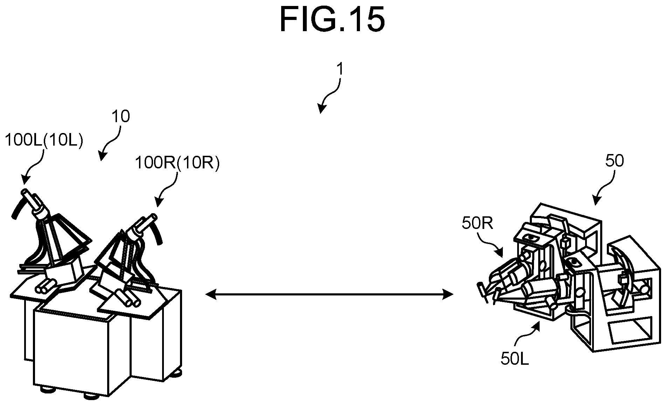

FIG. 15 is a schematic diagram for describing outlines of the medical arm system 1 according to the second embodiment. As illustrated in FIG. 15, the medical arm system 1 is provided with a master apparatus 10 and slave apparatuses 50 (50R and 50L). The master apparatus 10 is provided with the operation apparatus 100R for the right hand and the operation apparatus 100L for the left hand. Also, as the slave apparatuses 50, two apparatuses, i.e., the slave apparatus 50R for the right hand and the slave apparatus 50L for the left hand are provided. The slave apparatuses 50R and 50L correspond to the operation apparatuses 100R and 100L, respectively. Although illustration is omitted, the monitor 30 which displays the operative field is connected to the master apparatus 10 as well as FIG. 1.

The medical arm system 1 employs, as an example, bilateral control. Note that the master apparatus 10 and the slave apparatus 50 are connected to each other by an arbitrary communication method. For example, the master apparatus 10 and the slave apparatus 50 are connected to each other by wire communication or wireless communication. Also, for example, the master apparatus 10 and the slave apparatus 50 may be configured to carry out the communication directly or may be configured to carry out the communication via a network (or another apparatus).

The user holds the operation apparatuses 100R and 100L with the right hand and the left hand, respectively. In this state, the user operates the operation apparatuses 100R and 100L while watching the monitor 30, which displays the operative field. The user may remotely operate the positions or the directions of the operation tools attached to the slave apparatuses 50R and 50L or carry out holding actions by the respective operation tools by displacing the positions and the directions of the respective operation apparatuses 100R and 100L.

### 2.2. Details of Slave Apparatuses

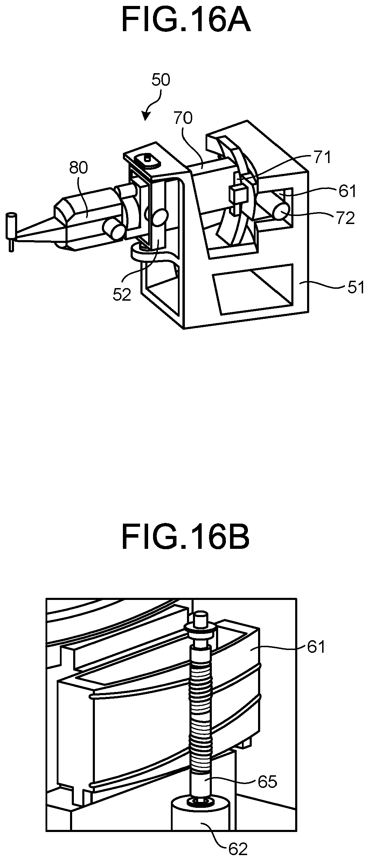

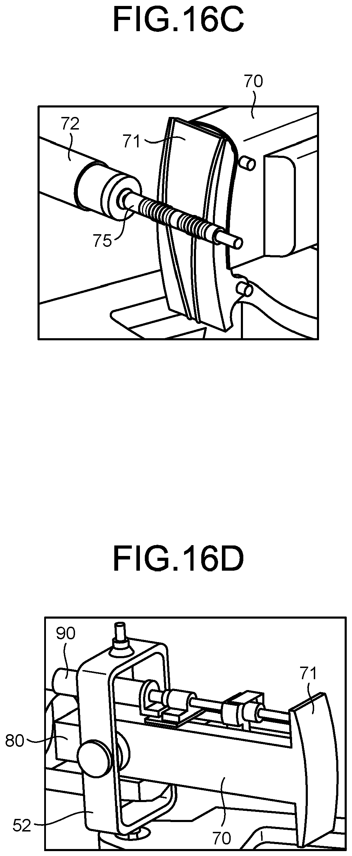

Hereinafter, the slave apparatus 50 according to the second embodiment will be described in more detail with reference to FIG. 16A to FIG. 18E. FIG. 16A is a schematic diagram illustrating an external configuration example of the slave apparatus 50 according to the second embodiment. FIG. 16B is an explanatory diagram for describing the first cable speed reducer 61 of the slave apparatus 50 according to the second embodiment. FIG. 16C is an explanatory diagram for describing the second cable speed reducer 71 of the slave apparatus 50 according to the second embodiment. FIG. 16D is an explanatory diagram for describing a slide mechanism 90 of the slave apparatus 50 according to the

### second embodiment.

As illustrated in FIG. 16A, in the slave apparatus 50 according to the second embodiment, each of the base 51, the first unit 60, the first cable speed reducer 61, the first motor 62, the second unit 70, the second cable speed reducer 71, the second motor 72, and the end effector unit 80 is covered with exterior in each unit. The gimbal 52 supports the second unit 70 from outside of the exterior. The entire configuration of the slave apparatus 50 according to the second embodiment is basically similar to that of the slave apparatus 50 according to the first embodiment.

As illustrated in FIG. 16B and FIG. 16C, each of the cable speed reducers such as the first cable speed reducer 61, which is used in the arc motions about the Phi axis, and the second cable speed reducer 71, which is used in the arc motions about the Theta axis, can enhance rigidity by using two or more cables. Also, as illustrated in FIG. 16B and FIG. 16C, moment force by the cables that acts on the input-side capstan can be reduced by reversing the directions of the winding directions of the two cables on the input-side capstan (power transmission part 65, 75). The cable is preferred to be wound around the input-side capstan plural times, and part of the wound part may be fixed by soldering or the like so as not to cause displacing with respect to the input-side capstan. In order to enhance the rigidity of each of the cable speed reducers, it is preferred that certain tension is configured to act on the cable by a coil spring or the like.

As illustrated in FIG. 16D, the end effector unit 80 can be also enabled to carry out slide motion in the R-axis direction by providing the slide mechanism 90 such as a ball screw, which carries out linear motion, between the second unit 70 and the end effector unit 80. In the example illustrated in FIG. 16D, the slide mechanism 90 is provided in the exterior of the second unit 70.

Note that, as the actuators which cause the slave apparatus 50 according to the second embodiment to carry out rotary actions about the axes of the Phi axis, the Theta axis, the Roll axis, the Pitch axis, the Yaw axis, and the Grip axis, rotary-type ultrasonic motors using piezoelectric elements, oil hydraulic rotary motors, electrostatic motors, etc. may be used other than electromagnetic rotary motors.

Also, as an actuator for causing the end effector unit 80 to carry out slide motion in the direction of the R axis, a rotary-type ultrasonic motor using piezoelectric elements, an oil hydraulic rotary motor, an electrostatic motor, a direct ultrasonic motor using piezoelectric elements, an oil hydraulic direct actuator (power cylinder), a polymeric actuator, a voice coil, an electromagnetic linear motor, or the like may be used other than an electromagnetic rotary motor.

Also, each of the above described actuators may be provided with a position detecting device and/or an emergency-stop brake such as a gear-type speed reducer, a harmonic-gear speed reducer, a planetary-gear speed reducer, a paradox planetary-gear speed reducer, a cable speed reducer, a traction speed reducer, a ball screw, a sliding screw, a speed reducer such as a worm gear, a magnetic encoder, an optical encoder, a potentiometer, etc.

Furthermore, the base 51 of the slave apparatus 50 may be in the upper side with respect to the ground. In other words, the top/bottom of the slave apparatus 50 can be inverted. Also, the base 51 per se may be coupled to an action device such as another manipulator or a direct acting stage.

Also, as a guide method of the Phi axis, the direction of motion can be regulated by using the arc guide 64 as well as the first embodiment so that the first unit 60 can smoothly carry out arc motions about the Phi axis. Other than that, the direction of motion may be regulated by using another guide method such as use of a cam follower for the first unit 60.

Also, as a guide method of the Theta axis, the direction of motion can be regulated by using the cam follower 63 as well as the first embodiment so that the second unit 70 can smoothly carry out arc motions about the Theta axis. Other than that, the direction of motion may be regulated by using another guide method such as use of an arc guide for the second unit 70.

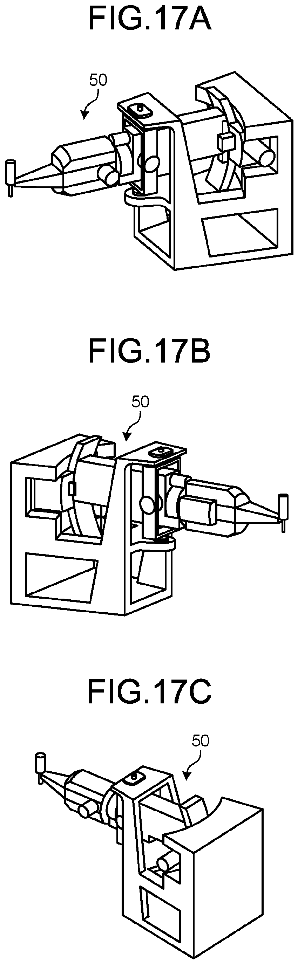

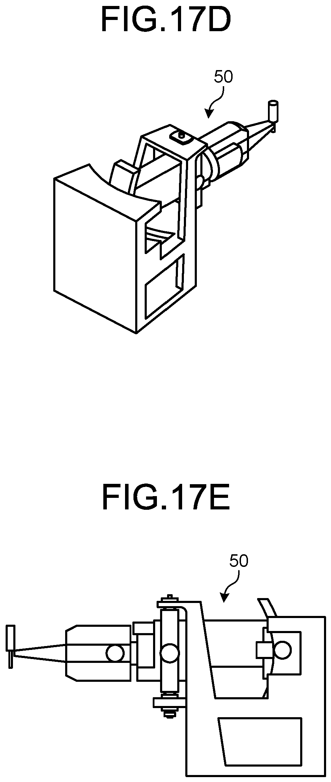

FIG. 17A to FIG. 17E are image views illustrating states of the slave apparatus 50 according to the second embodiment viewed from various angles. FIG. 17A is a first perspective view of the slave apparatus 50 according to the second embodiment. FIG. 17B is a second perspective view of the slave apparatus 50 according to the second embodiment. FIG. 17C is a third perspective view of the slave apparatus 50 according to the second embodiment. FIG. 17D is a fourth perspective view of the slave apparatus 50 according to the second embodiment. FIG. 17E is a lateral view of the slave apparatus 50 according to the second embodiment.

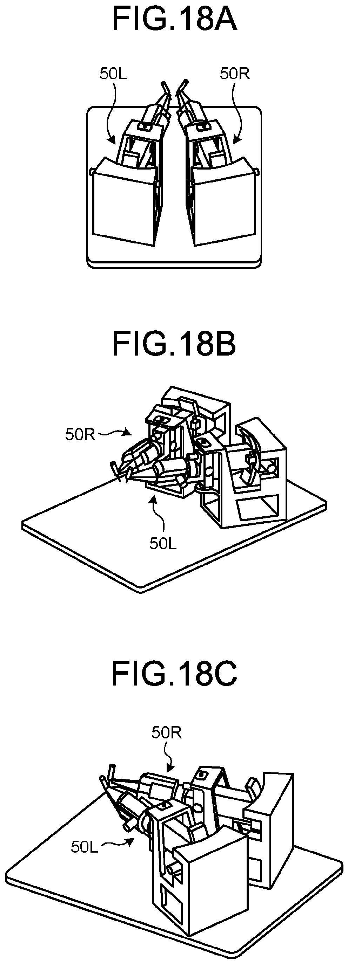

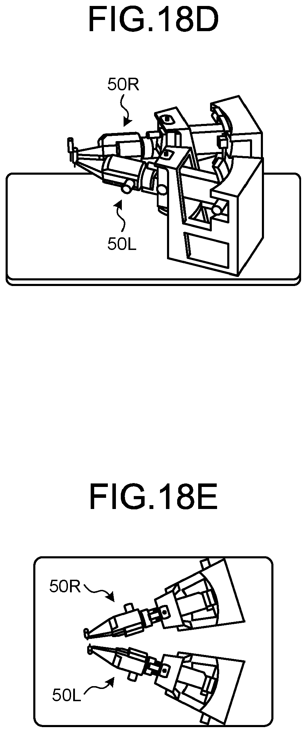

FIG. 18A to FIG. 18E are image views of cases in which two apparatuses, i.e., the slave apparatuses 50 (50R and 50L) according to the second embodiment are juxtaposed and used. FIG. 18A is a rear view of the case in which the two apparatuses, i.e., the slave apparatuses 50 (50R and 50L) according to the second embodiment are juxtaposed and used. FIG. 18B is a first perspective view of the case in which the two apparatuses, i.e., the slave apparatuses 50 (50R and 50L) according to the second embodiment are juxtaposed and used. FIG. 18C is a second perspective view of the case in which the two apparatuses, i.e., the slave apparatuses 50 (50R and 50L) according to the second embodiment are juxtaposed and used. FIG. 18D is a lateral view of the case in which the two apparatuses, i.e., the slave apparatuses 50 (50R and 50L) according to the second embodiment are juxtaposed and used. FIG. 18E is a top view of the case in which the two apparatuses, i.e., the slave apparatuses 50 (50R and 50L) according to the second embodiment are juxtaposed and used.

### 3. Conclusion

A goal of a bilateral control system is "realization of the sensation that an operator is in the world of a 1/N scale and is carrying out physical operations". The term "1/N" referred to herein means work in small space in which it is difficult for a person to carry out work such as 1/3 or 1/10. An operation in a scale of 1/10 means that, when the operator operates a master arm by 10 mm, a slave arm moves by 1 mm. In order to achieve the above described objective, "accuracy that works in the world of a scale of 1/N" and "high direct operating sensations" are important. When these two elements are converted to the performance required for the arms are "high position and force resolution" for the former one and "high natural frequency" and "sufficient movable ranges" for the latter one. In a mechanism structure (joint structure) of a conventional slave arm, when movable ranges are expanded to increase the degrees of freedom, the mechanism size inevitably increases, and it is therefore difficult to realize a high natural frequency at the same time. Also, in order to realize the precision of both positions and force, for example, the joints of the mechanism are required to be free from backlash and high back-drivability is also required in addition.

To avoid the operator from feeling intervention of a robot in a high frequency range in a bilateral control system having a master arm and a slave arm, arms with a high natural frequency which can be stably controlled in the high frequency range are required. The present disclosure relates to a mechanism structure (oar mechanism) suitable for a slave arm which does not require a wide translational movable range and has following advantages. First, by supporting the vicinity of the gravity center of the movable part with the gimbal, the gravity compensating torque required for the motor is reduced. In addition, since motor torque is disposed at the position distant from the rotation center, high speed reducing ratios can be obtained even when the wire speed reducing structures are used. Therefore, the direction of the mechanism can be controlled at high precision with low torque. Furthermore, since the point of support and the point of effort are distant from each other, the natural frequency of the mechanism can be easily increased. Moreover, the conventional arm structure has to enlarge the motor at the root of the arm to carry out gravity compensation. On the other hand, in the present disclosure, the motor at the root of the arm can be downsized since gravity compensation is carried out by the gimbal. For example, in the present disclosure, a precision speed reducer having a large diameter is not required to be used at the root part of the arm unlike the conventional arm structure, and a cable speed reducer can be used. According to the present disclosure, a polar-coordinate-type manipulator suitable for precision operations can be provided.

The preferred embodiments of the present disclosure have been described in detail hereinabove with reference to the accompanying drawings, but the technical scope of the present disclosure is not limited to such examples. It is apparent that a person having ordinary knowledge in the technical field of the present disclosure can conceive of various changes or modifications within the scope of the technical idea described in the claims, and it is understood that the changes or modifications also naturally belong to the technical scope of the present disclosure.

Furthermore, the effects described in the present specification are merely illustrative or exemplary and are not limitative. That is, the technology according to the present disclosure can exhibit other effects obvious to those skilled in the art from the description of the present specification in addition to or in place of the above described effects.

The present technique can also employ following configurations.
(1) A medical arm system comprising:
   an operation apparatus operated by an operator; and
   an arm apparatus remotely operated in response to an operation of the operator with respect to the operation apparatus, wherein
   the arm apparatus has
   a base,
   a first unit connected to the base,
   a second unit connected to the first unit,
   a gimbal connected to the base and supporting the second unit, and
   an end effector unit connected to the second unit and provided with an operation tool to contact a patient,
   the first unit moves the second unit in a direction of at least one axis with respect to the base, and
   the second unit is interlocked with the first unit in a state supported by the gimbal and moves the end effector unit in the direction of the at least one axis.
(2) The medical arm system according to (1), wherein the gimbal supports rotation in two axes.
(3) The medical arm system according to (1) or (2), wherein the second unit interlocks with the first unit and carries out an arc motion about a first axis of the gimbal to cause the end effector unit to carry out an arc motion about the first axis.
(4) The medical arm system according to any one of (1) to (3), wherein the second unit carries out an arc motion about a second axis of the gimbal, which is not in a direction of movement caused by interlocking with the first unit, to cause the end effector unit to carry out an arc motion about the second axis.
(5) The medical arm system according to any one of (1) to (4), wherein the end effector unit carries out a slide motion in a long axis direction of the second unit in a state that the second unit is supported by the gimbal.
(6) The medical arm system according to any one of (1) to (5), wherein the end effector unit carries out a rotation motion about a long axis of the second unit in a state that the second unit is supported by the gimbal.
(7) The medical arm system according to any one of (1) to (6), wherein the operation tool moves in a direction of at least one axis with respect to the end effector unit in a state that the second unit is supported by the gimbal.
(8) The medical arm system according to any one of (1) to (7), wherein a shape of the gimbal is any of a center hollow shape, a U shape, and an L shape.
(9) The medical arm system according to any one of (1) to (8), wherein the arm apparatus carries out an action in seven axes in total including a three-axis operation-tool-position changing action in a Phi axis, a Theta axis, and an R axis, a three-axis operation-tool rotating action in a Yaw axis, a Pitch axis, and a Roll axis, and an operation-tool opening/closing action in a Grip axis.
(10) The medical arm system according to any one of (1) to (9), wherein the arm apparatus has a root provided with a cable speed reducer.
(11) The medical arm system according to (10), wherein the cable speed reducer uses at least two cables.
(12) The medical arm system according to (11), wherein
   the cable speed reducer has an input-side capstan coupled to a motor, the at least two cables being wound around the input-side capstan, and
   directions of winding the at least two cables around the input-side capstan are opposite directions.
(13) The medical arm system according to (10), wherein a speed reducing ratio of the cable speed reducer is r/R, in a case where a radius of an input-side capstan about an axis of a motor is r and a radius of an output-side capstan of the cable speed reducer about an axis of the gimbal is R.
(14) The medical arm system according to any one of (1) to (13), wherein the arm apparatus carries out an arc motion about an axis of the gimbal and regulates a motion direction by using at least one of an arc guide and a cam follower.
(15) An arm apparatus comprising:
   a base;
   a first unit connected to the base;
   a second unit connected to the first unit;
   a gimbal connected to the base and supporting the second unit; and
   an end effector unit connected to the second unit and provided with an end effector to act on an object, wherein
   the first unit moves the second unit in a direction of at least one axis with respect to the base, and
   the second unit is interlocked with the first unit in a state supported by the gimbal and moves the end effector unit in the direction of the at least one axis.
(16) An actuation method of a master/slave system including a master apparatus operated by an operator and a slave apparatus remotely operated in response to an operation of the operator with respect to the master apparatus, wherein
   the master/slave system controls the slave apparatus based on input of the operator with respect to the master apparatus, and
   the slave apparatus interlocks and moves a first unit and a second unit to move an operation-tool attachable end effector unit in a direction of at least one axis, the first unit being connected to a base, the second unit being connected to the first unit in the direction of the at least one axis with respect to the base, the second unit being supported by a gimbal, the end effector unit being connected to the second unit.

### Reference Signs List

1 MEDICAL ARM SYSTEM
10, 10R, 10L MASTER APPARATUS
100, 100R, 100L OPERATION APPARATUS
30 MONITOR
50, 50A, 50B, 50C, 50R, 50L SLAVE APPARATUS (ARM APPARATUS)
51 BASE
52, 52A, 52B, 52C GIMBAL
60 FIRST UNIT
61 FIRST CABLE SPEED REDUCER
62 FIRST MOTOR
70, 70A, 70B, 70C SECOND UNIT
71 SECOND CABLE SPEED REDUCER
72 SECOND MOTOR
80, 80A, 80B END EFFECTOR UNIT
81 ARM PART
82 GRIPPER

## Claims

1. A medical arm system comprising:
an operation apparatus operated by an operator; and
an arm apparatus remotely operated in response to an operation of the operator with respect to the operation apparatus, wherein
the arm apparatus has
a base,
a first unit connected to the base,
a second unit connected to the first unit,
a gimbal connected to the base and supporting the second unit, and
an end effector unit connected to the second unit and provided with an operation tool to contact a patient,
the first unit moves the second unit in a direction of at least one axis with respect to the base, and
the second unit is interlocked with the first unit in a state supported by the gimbal and moves the end effector unit in the direction of the at least one axis.

2. The medical arm system according to claim 1, wherein the gimbal supports rotation in two axes.

3. The medical arm system according to claim 1, wherein the second unit interlocks with the first unit and carries out an arc motion about a first axis of the gimbal to cause the end effector unit to carry out an arc motion about the first axis.

4. The medical arm system according to claim 1, wherein the second unit carries out an arc motion about a second axis of the gimbal, which is not in a direction of movement caused by interlocking with the first unit, to cause the end effector unit to carry out an arc motion about the second axis.

5. The medical arm system according to claim 1, wherein the end effector unit carries out a slide motion in a long axis direction of the second unit in a state that the second unit is supported by the gimbal.

6. The medical arm system according to claim 1, wherein the end effector unit carries out a rotation motion about a long axis of the second unit in a state that the second unit is supported by the gimbal.

7. The medical arm system according to claim 6, wherein the operation tool moves in a direction of at least one axis with respect to the end effector unit in a state that the second unit is supported by the gimbal.

8. The medical arm system according to claim 1, wherein a shape of the gimbal is any of a center hollow shape, a U shape, and an L shape.

9. The medical arm system according to claim 1, wherein the arm apparatus carries out an action in seven axes in total including a three-axis operation-tool-position changing action in a Phi axis, a Theta axis, and an R axis, a three-axis operation-tool rotating action in a Yaw axis, a Pitch axis, and a Roll axis, and an operation-tool opening/closing action in a Grip axis.

10. The medical arm system according to claim 1, wherein the arm apparatus has a root provided with a cable speed reducer.

11. The medical arm system according to claim 10, wherein the cable speed reducer uses at least two cables.

12. The medical arm system according to claim 11, wherein
the cable speed reducer has an input-side capstan coupled to a motor, the at least two cables being wound around the input-side capstan, and
directions of winding the at least two cables around the input-side capstan are opposite directions.

13. The medical arm system according to claim 10, wherein a speed reducing ratio of the cable speed reducer is r/R, in a case where a radius of an input-side capstan about an axis of a motor is r and a radius of an output-side capstan of the cable speed reducer about an axis of the gimbal is R.

14. The medical arm system according to claim 1, wherein the arm apparatus carries out an arc motion about an axis of the gimbal and regulates a motion direction by using at least one of an arc guide and a cam follower.

15. An arm apparatus comprising:
a base;
a first unit connected to the base;
a second unit connected to the first unit;
a gimbal connected to the base and supporting the second unit; and
an end effector unit connected to the second unit and provided with an end effector to act on an object, wherein
the first unit moves the second unit in a direction of at least one axis with respect to the base, and
the second unit is interlocked with the first unit in a state supported by the gimbal and moves the end effector unit in the direction of the at least one axis.

16. An actuation method of a master/slave system including a master apparatus operated by an operator and a slave apparatus remotely operated in response to an operation of the operator with respect to the master apparatus, wherein
the master/slave system controls the slave apparatus based on input of the operator with respect to the master apparatus, and
the slave apparatus interlocks and moves a first unit and a second unit to move an operation-tool attachable end effector unit in a direction of at least one axis, the first unit being connected to a base, the second unit being connected to the first unit in the direction of the at least one axis with respect to the base, the second unit being supported by a gimbal, the end effector unit being connected to the second unit.
